# EUROPEAN PATENT APPLICATION

(11) **EP 3 064 202 A1**
(43) Date of publication of application: **07.09.2016**
(21) Application number: 14858331.3
(22) Date of filing: 22.10.2014
(51) Int. Cl.: A61K 31/197, A61P 7/00, A61P 7/06, A61P 25/00, A61P 25/14, A61P 43/00

(54) **FRATAXIN ENHANCER**

(30) Priority: 29.10.2013 JP 2013223929
(71) Applicant: Tokyo University of Agriculture, Tokyo 156-8502 (JP); SBI Pharmaceuticals Co., Ltd., Tokyo 106-6020 (JP)
(72) Inventor: CHIBAZAKURA, Taku, Tokyo 156-8502 (JP); TANAKA, Tohru, Tokyo 106-6020 (JP); ABE, Fuminori, Tokyo 106-6020 (JP); NAKAJIMA, Motowo, Tokyo 106-6020 (JP); KAMIYA, Atsuko, Tokyo 106-6020 (JP); HAGA, Naomi, Tokyo 106-6020 (JP); TAKAHASHI, Kiwamu, Tokyo 106-6020 (JP)
(74) Representative: Arends, William Gerrit
(86) International application number: PCT/JP2014/078121
(87) International publication number: WO 2015/064447

(57) **Abstract**

The object of the present invention to provide an agent effective for treating and preventing e.g. a disease caused by reduction in frataxin production. The present invention provides a frataxin enhancer comprising 5-aminolevulinic acid (ALA) or a derivative thereof or salts thereof, as well as a therapeutic and/or prophylactic agent for a disease caused by reduction in frataxin production.

## Description

### Technical Field

The present invention relates to a frataxin enhancer, as well as a therapeutic and/or prophylactic agent for a disease caused by reduction in frataxin production. In particular, the present invention relates to a frataxin enhancer comprising 5-aminolevulinic acid (ALA) or a derivative thereof or salts thereof, as well as a therapeutic and/or prophylactic agent for a disease caused by reduction in frataxin production.

### Background Art

Frataxin is an iron-binding protein that exists in mitochondria, and is known to bear a role as an iron chaperone protein in restoring the aconitase activity of the citric acid circuit that has been lowered by reactive oxygen species. Frataxin is also known to be responsible for supplying iron to iron-sulfur clusters. Iron-sulfur clusters exist as e.g. the activity center of aconitase which is an enzyme that converts citric acid into isocitric acid in the mitochondrial TCA circuit or as a component of mitochondrial electron transport chain complex I (Non-Patent Literatures 1 and 2).

In this manner, frataxin is an extremely important factor in vital activity that is related to e.g. homeostasis of iron *in vivo,* and reduction in the amount of frataxin protein *in vivo* has a great impact on the sustenance of vital activity. A known disease caused by reduction in frataxin production is Friedreich's ataxia (FRDA or FA). FRDA, the most common hereditary ataxia among Caucasians, is a disease caused by the lack of mRNA encoding frataxin. Progressive spinocerebellar neurodegeneration occurs when frataxin lacks *in vivo,* and as a result, symptoms such as incoordination of gait and hand movement, tangled words, and weakness and sensory loss of muscles emerge. FRDA may also be accompanied by extraneural scoliosis, myocardiopathy, and diabetes. Although antioxidants or iron chelators have been employed for treatment of FRDA, these treatment methods are merely palliative therapies for symptoms accompanying reduction in frataxin and do not enhance frataxin itself as fundamental therapy.

Application of histone deacetylase inhibitor (HDAC inhibitor) has been proposed as an attempt to enhance the amount of frataxin protein *in vivo* (Patent Literature 1). However, HDAC is a protein involved in various cell functions including transcription control. Moreover, there are many isozymes of HDAC (at least 8 types for humans), the function of each of which is thought to be different. Accordingly, various side effects are predicted from enhancement of frataxin with HDAC inhibitor and practical application has not been achieved thus far.

### Citation List

[Patent Literature 1] Japanese Translation of PCT International Application Publication No. 2009-515887

[Non-Patent Literature 1] Science 305, 242-245 (2004)
[Non-Patent Literature 2] PNAS 102, 5987-5991 (2005)

### Summary of the Invention

### Problems to be Solved by the Invention

As described above, even though a demand for an agent that enhances the expression of frataxin protein that can be practically used for treating and/or preventing a disease caused by reduction in frataxin has been present for quite some time, there are still unsolved needs.

### Means for Solving the Problems

It is known that when 5-aminolevulinic acids (ALAs) are administered *in vivo,* it is incorporated into the heme metabolic pathway and intermediate metabolic product protoporphyrin IX (PpIX) is produced. It has been reported that in an experiment employing mouse fibroblasts (LMTK cells), the amount of frataxin protein is decreased by addition of protoporphyrin IX (Blood, May, 2002; 99:3813-22). In other words, in light of the aforementioned report, it has been predicted that the amount of frataxin protein will be decreased when ALAs which are precursors of PpIX are administered to cells and/or *in vivo.*

However, in the process of closely investigating a group of proteins associated with the heme biosynthetic pathway when ALAs are administered to cells, the present inventors found contrary to conventional prediction that the amount of frataxin protein in cells was increased. Based on this surprising finding, the present inventors came to complete the present invention.

ALAs are amino acids that exist *in vivo,* which are known to be highly safe for the human body. In other words, *in vivo* administration of ALAs has almost no side effects, and practical use of the present invention is extremely high.

In other words, the present invention relates to a frataxin enhancer comprising a compound represented by the following Formula (I): 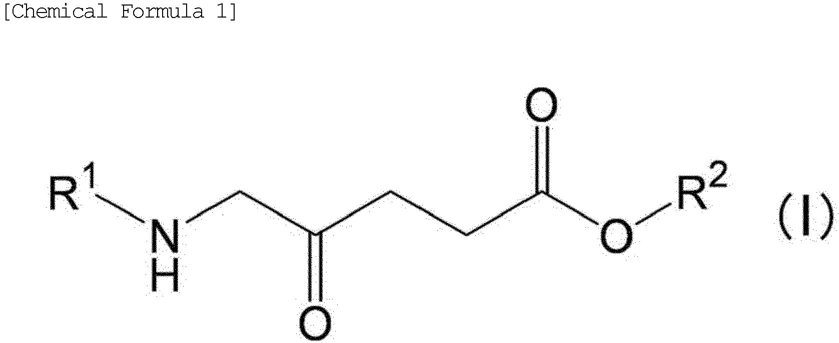 (wherein R¹ represents a hydrogen atom or an acyl group, and R² represents a hydrogen atom, a linear or branched alkyl group, a cycloalkyl group, an aryl group, or an aralkyl group.)
or a salt thereof.

In one aspect, the compound represented by the above Formula (I) may be those wherein R¹ may be selected from the group consisting of a hydrogen atom, an alkanoyl group having 1 - 8 carbons, and an aroyl group having 7 - 14 carbons, and R² may be selected from the group consisting of a hydrogen atom, a linear or branched alkyl group having 1 - 8 carbons, a cycloalkyl group having 3 - 8 carbons, an aryl group having 6 - 14 carbons, and an aralkyl group having 7 - 15 carbons.

Moreover, in one aspect, the compound represented by the above Formula (I) may be those wherein R¹ and R² may be hydrogen atoms.

In another aspect, the present invention relates to a therapeutic and/or prophylactic agent for a disease caused by reduction in frataxin production comprising a compound represented by the above Formula (I) or a salt thereof.

Moreover, in one aspect, said "disease caused by reduction in frataxin production" may be Friedreich's ataxia.

In another aspect, the present invention relates to a method for enhancing frataxin, characterized in administering to a subject a therapeutically effective amount of a compound represented by the above Formula (I) or a salt thereof.

In another aspect, the present invention relates to a method of treating and/or preventing a disease caused by reduction in frataxin production, characterized in administering to a subject a compound represented by the above Formula (I) or a salt thereof.

In another aspect, the present invention relates to the use of a compound represented by the above Formula (I) or a salt thereof for manufacturing a frataxin enhancer.

In another aspect, the present invention relates to the use of a compound represented by the above Formula (I) or a salt thereof for manufacturing a therapeutic and/or prophylactic agent for a disease caused by reduction in frataxin production.

### Brief Description of the Drawings

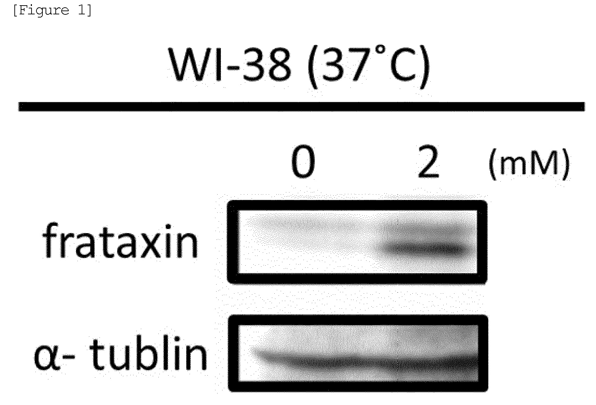

Figure 1 compares the amount of frataxin protein expressed between WI-38 cells cultured in a medium comprising ALA and WI-38 cells cultured in a medium that does not comprise ALA.

### Description of Embodiments

"Enhancement of frataxin" as used herein encompasses e.g. increasing the amount of mRNA encoding frataxin that is transcripted *in vivo*/*in vitro* in a cell, or increasing the amount of frataxin protein expressed *in vivo*/*in vitro* in a cell. The *in vivo* site where frataxin is increased by the present invention is not limited to any particular cell, tissue, or organ.

A "disease caused by reduction in frataxin production" as used herein broadly encompasses e.g. diseases that are directly/indirectly caused by reduction in the *in vivo* amount of frataxin protein expressed having normal activity, such as absence of the gene encoding frataxin, mutation of the gene encoding frataxin, abnormality in transcription/translation of the gene encoding frataxin, and abnormality in a part of the pathway related to the expression of frataxin protein.

According to the present invention, because the *in vivo* frataxin production can be increased, when the frataxin enhancer of the present invention is administered to a subject, *in vivo* iron utilization efficiency rises along with the increase in frataxin. In other words, according to the present invention, not only diseases caused directly by reduction in frataxin can be treated and/or prevented, but diseases caused by abnormality in *in vivo* iron utilization efficiency, as well as diseases caused by the presence of excess iron *in vivo* can also be treated and/or prevented. A disease caused by abnormality in *in vivo* iron utilization efficiency can include anemia of chronic disease (ACD). An example of a disease caused by the presence of excess iron *in vivo* can include iron overload due to blood transfusion.

ALAs as used herein refer to an ALA or a derivative thereof, or salts thereof.

ALA as used herein means a 5-aminolevulinic acid. ALA is also referred to as δ-aminolevulinic acid, and is a type of amino acid.

The compound represented by the following Formula (I) can be exemplified as an example of an ALA derivative. In Formula (I), R¹ represents a hydrogen atom or an acyl group, and R represents a hydrogen atom, a linear or branched alkyl group, a cycloalkyl group, an aryl group, or an aralkyl group. Note that in Formula (I), ALA corresponds to when R¹ and R² are hydrogen atoms.

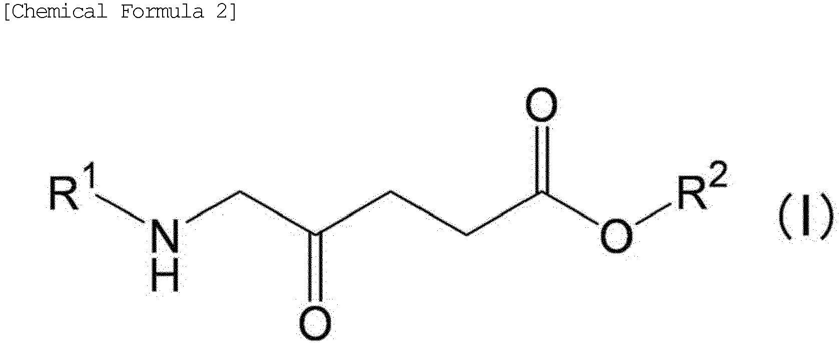

ALAs may act as an active ingredient *in vivo* in the form of the ALA of Formula (I) or a derivative thereof, and can also be administered as a prodrug (precursor) that is degradated by an *in vivo* enzyme.

The acyl group in R¹ of Formula (I) can include a linear or branched alkanoyl group having 1 - 8 carbons such as formyl, acetyl, propionyl, butyryl, isobutyryl, valeryl, isovaleryl, pivaloyl, hexanoyl, octanoyl, and benzylcarbonyl groups, and an aroyl group having 7 - 14 carbons such as benzoyl, 1-naphthoyl, and 2-naphthoyl groups.

The alkyl group in R² of Formula (I) can include a linear or branched alkyl group having 1 - 8 carbons such as methyl, ethyl, propyl, isopropyl, butyl, isobutyl, sec-butyl, tert-butyl, pentyl, isopentyl, neopentyl, hexyl, heptyl, and octyl groups.

The cycloalkyl group in R² of Formula (I) can include a cycloalkyl group having 3 - 8 carbons which may be saturated or have partially unsaturated bonds, such as cyclopropyl, cyclobutyl, cyclopentyl, cyclohexyl, cycloheptyl, cyclooctyl, cyclododecyl, and 1-cyclohexenyl groups.

The aryl group in R² of Formula (I) can include an aryl group having 6 - 14 carbons such as phenyl, naphthyl, anthryl, and phenanthryl groups.

The aralkyl group in R² of Formula (I) can be exemplified with the same aryl groups as above as the aryl moiety and the same alkyl groups as above as the alkyl moiety, and can specifically include an aralkyl group having 7 - 15 carbons such as benzyl, phenethyl, phenylpropyl, phenylbutyl, benzhydryl, trityl, naphthylmethyl, and naphthylethyl groups.

Preferred ALA derivatives include compounds where R¹ is a formyl group, an acetyl group, a propionyl group, a butyryl group, and the like. Moreover, preferred ALA derivatives also include compounds where the above R² is a methyl group, an ethyl group, a propyl group, a butyl group, a pentyl group, and the like. Moreover, preferred ALA derivatives also include compounds where the combination of the above R¹ and R² is each combination of (formyl and methyl), (acetyl and methyl), (propionyl and methyl), (butyryl and methyl), (formyl and ethyl), (acetyl and ethyl), (propionyl and ethyl), and (butyryl and ethyl).

Among ALAs, a salt of an ALA or a derivative thereof can include a pharmacologically acceptable acid addition salt, a metal salt, an ammonium salt, an organic amine addition salt, and the like. Acid addition salts can be exemplified by e.g. each of inorganic acid salts such as a hydrochloride salt, a hydrobromide salt, a hydroiodide salt, a phosphate salt, a nitrate salt, and a sulfate salt, and each of organic acid addition salts such as a formate salt, an acetate salt, a propionate salt, a toluenesulfate salt, a succinate salt, an oxalate salt, a lactate salt, a tartrate salt, a glycolate salt, a methanesulfonate salt, a butyrate salt, a valerate salt, a citrate salt, a fumarate salt, a maleate salt, and a malate salt. Metal salts can be exemplified by each of alkali metal salts such as a lithium salt, a sodium salt, and a potassium salt, each of alkaline earth metal salts such as a magnesium salt and a calcium salt, and each of metal salts such as aluminum and zinc. Ammonium salts can be exemplified by alkyl ammonium salts such as an ammonium salt and a tetramethylammonium salt. Organic amine salts can be exemplified by each of salts such as a triethylamine salt, a piperidine salt, a morpholine salt, and a toluidine salt. Note that these salts can also be employed as a solution at the time of use.

Among the above ALAs, the most favorable are ALA and various esters such as an ALA methyl ester, an ALA ethyl ester, an ALA propyl ester, an ALA butyl ester, and an ALA pentyl ester, as well as hydrochloride salts, phosphate salts, and sulfate salts thereof. In particular, ALA hydrochloride salts and ALA phosphate salts can be exemplified as particularly favorable.

The above ALAs can be manufactured by e.g. well-known methods such as chemical synthesis, production by microorganisms, and production by enzymes. Moreover, the above ALAs may also form a hydrate or a solvate, and ALAs can be employed alone or in an appropriate combination of two or more.

When the above ALAs are to be prepared as an aqueous solution, attention must be paid so that the aqueous solution will not become alkaline in order to prevent degradation of ALAs. In the case it becomes alkaline, degradation can be prevented by removing oxygen.

The administration route of the frataxin enhancer of the present invention or a therapeutic and/or prophylactic agent for a disease caused by reduction in frataxin production can include oral administration including sublingual administration, or parenteral administration such as administration by inhalation, intravenous administration including infusion, transdermal administration by e.g. poultices, suppositories, or administration by forced enteral nutrition employing a nasogastric tube, a nasointestinal tube, a gastrostomy tube, or an enterostomy tube, and the like.

The dosage form of the frataxin enhancer of the present invention or a therapeutic and/or prophylactic agent for a disease caused by reduction in frataxin production can be appropriately determined according to the above administration route, and can include injections, infusions, tablets, capsules, fine granules, powders, liquids, solutions dissolved in e.g. a syrup, poultices, suppositories, and the like.

In order to prepare the frataxin enhancer of the present invention, or a therapeutic and/or prophylactic agent for a disease caused by reduction in frataxin production, a carrier, an excipient, a diluent, an additive, a disintegrant, a binder, a coating agent, lubricant, a gliding agent, a lubricant, a flavoring agent, a sweetening agent, a solubilizer, a solvent, a gelling agent, and a nutrient etc. which may be pharmacologically acceptable can be added as necessary, specific examples of which can include water, saline, animal fat and oil, vegetable oil, lactose, starch, gelatin, crystalline cellulose, gum, talc, magnesium stearate, hydroxypropylcellulose, polyalkylene glycol, polyvinyl alcohol, and glycerin.

The administration amount, frequency, and duration of the frataxin enhancer of the present invention, or a therapeutic and/or prophylactic agent for a disease caused by reduction in frataxin production will vary depending on the age, weight, and symptoms etc. of the subject. The dosage of ALAs can include 0.01 mmol - 25 mmol/day, preferably 0.025 mmol - 7.5 mmol/day, more preferably 0.075 mmol - 5.5 mmol/day, and further preferably 0.2 mmol - 2 mmol/day (ALA molar equivalent) per adult. In particular, when employed as a prophylactic agent, it is desirable to continuously ingest a low dosage. Examples of the administration frequency can include one to multiple administration(s) per day or continuous administration by e.g. infusion. When employed as a therapeutic agent, the administration duration can be determined by a pharmacologist or a clinician skilled in the art by a known method based on e.g. the amount of the frataxin protein or a biochemical indicator related to frataxin *in vivo.* When employed as a prophylactic agent, the administration duration can be determined by a pharmacologist or a clinician skilled in the art by a known method based on e.g. the amount of the frataxin protein or a biochemical indicator related to frataxin, with observation so that aggravation of symptoms does not occur.

The frataxin enhancer of the present invention or a therapeutic and/or prophylactic agent for a disease caused by reduction in frataxin production can also be used in combination with other agents employed for improving symptoms accompanying reduction in frataxin production. For example, it can be employed together with an agent employed as a palliative therapy for Friedreich's ataxia. Examples of these agents can include an antioxidant or an iron chelator.

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, and shall not be construed as being limited to the Examples described herein.

### Examples

### 1. Materials and Methods

(i) Human normal fibroblast strain WI-38 was cultured in Dulbecco's modified Eagle medium (DMEM) comprising 10% fetal bovine serum (FBS) under 5% CO₂. (ii) 5-Aminolevulinic acid (ALA) was added at a final concentration of 2 mM to DMEM without FBS, and cells of (i) were cultured in said medium at 37°C for 2 hours to allow ALA to be incorporated. (iii) Then, the cells were transferred to DMEM containing 10% FBS and cultured at 37°C for 24 hours. (iv) The cells were collected by trypsin treatment, washed with phosphate buffered saline (PBS), and then suspended in SDS sample buffer (50 mM Tris-HCl, pH6.8, 2% SDS, 2.5%-mercaptoethanol, 10% glycerol, and 0.01% bromophenol blue), and heated at 100°C for 5 minutes to allow lysis. In this Example, the said cell lysate is referred to as "Sample 1."

As a negative control, a cell lysate was prepared by a preparation similar to Sample 1 except that in the above step (ii), cells were cultured in a medium that does not comprise ALA. In this Example, the said cell lysate is referred to as "Sample 2."

Samples 1 and 2 were subjected to 12% SDS-polyacrylamide electrophoresis, and Western blot analysis employing an anti-human frataxin antibody (from Santa Cruz Biotechnology, cat. #sc-25820) and an HRP-labeled anti-rabbit IgG secondary antibody (from GE Healthcare, cat. #NA934V) was performed by conventional means. As a positive control, Samples 1 and 2 were subjected to 12% SDS-polyacrylamide electrophoresis, and Western blot analysis employing an anti-human α-tubulin antibody (from Santa Cruz Biotechnology, cat. #sc-8035) and an HRP-labeled anti-mouse IgG secondary antibody (from GE Healthcare, cat. #NA931V) was performed by conventional means. The results of the Western blot analysis are shown in Figure 1.

### 2. Results

As shown in Figure 1, surprisingly, a clear increase in the amount of frataxin protein was seen in Sample 1 in which cells were cultured in a medium comprising ALA compared to Sample 2 in which cells were cultured in a medium that does not comprise ALA.

## Claims

1. A frataxin enhancer comprising a compound represented by the following Formula (I): 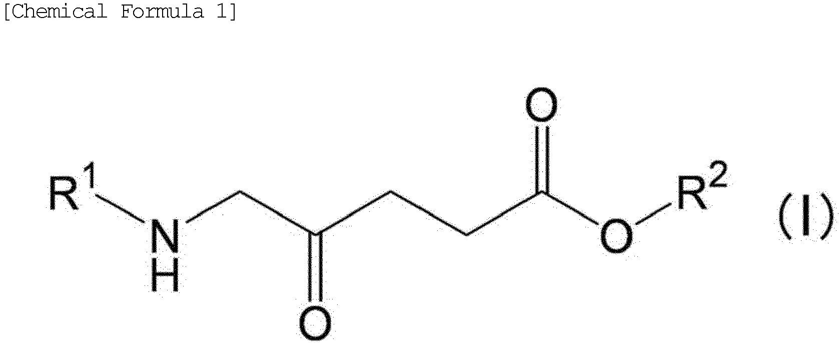 (wherein R¹ represents a hydrogen atom or an acyl group, and R² represents a hydrogen atom, a linear or branched alkyl group, a cycloalkyl group, an aryl group, or an aralkyl group.)
or a salt thereof.

2. The frataxin enhancer according to claim 1, **characterized in that** R¹ is selected from the group consisting of a hydrogen atom, an alkanoyl group having 1 - 8 carbons, and an aroyl group having 7 - 14 carbons, and
R² is selected from the group consisting of a hydrogen atom, a linear or branched alkyl group having 1 - 8 carbons, a cycloalkyl group having 3 - 8 carbons, an aryl group having 6 - 14 carbons, and an aralkyl group having 7 - 15 carbons.

3. The frataxin enhancer according to claim 1, **characterized in that** R¹ and R² are hydrogen atoms.

4. A therapeutic and/or prophylactic agent for a disease caused by reduction in frataxin production comprising a compound represented by the following Formula (I): 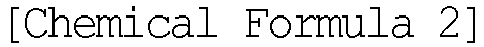 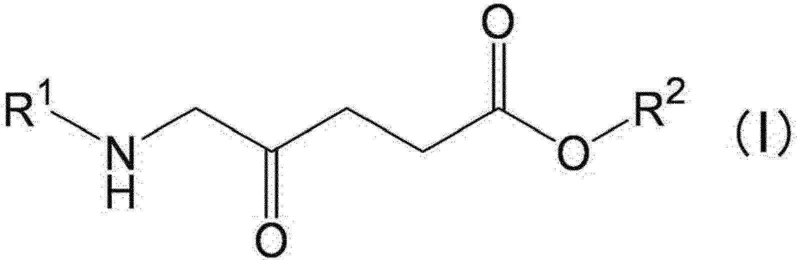 (wherein R¹ represents a hydrogen atom or an acyl group, and R² represents a hydrogen atom, a linear or branched alkyl group, a cycloalkyl group, an aryl group, or an aralkyl group.)
or a salt thereof.

5. The therapeutic and/or prophylactic agent for a disease caused by reduction in frataxin production according to claim 4, **characterized in that** said "disease caused by reduction in frataxin production" is Friedreich's ataxia.

6. A method for enhancing frataxin, **characterized in** administering to a subject a therapeutically effective amount of a compound represented by the following Formula (I): 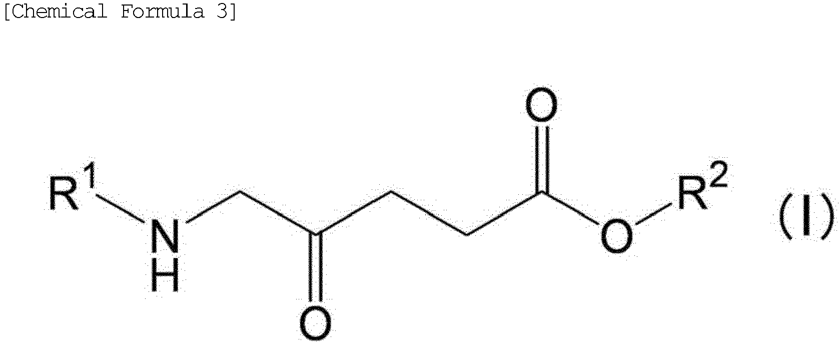 (wherein R¹ represents a hydrogen atom or an acyl group, and R² represents a hydrogen atom, a linear or branched alkyl group, a cycloalkyl group, an aryl group, or an aralkyl group.)
or a salt thereof.

7. A method of treating and/or preventing a disease caused by reduction in frataxin production, **characterized in** administering to a subject a compound represented by the following Formula (I): 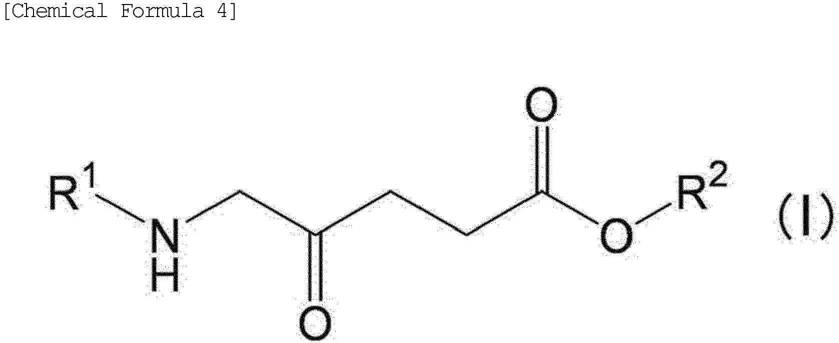 (wherein R¹ represents a hydrogen atom or an acyl group, and R² represents a hydrogen atom, a linear or branched alkyl group, a cycloalkyl group, an aryl group, or an aralkyl group.)
or a salt thereof.

8. The use of a compound represented by the following Formula (I): 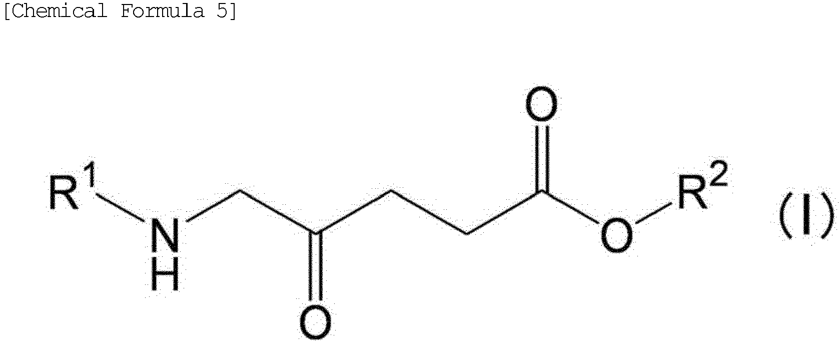 (wherein R¹ represents a hydrogen atom or an acyl group, and R² represents a hydrogen atom, a linear or branched alkyl group, a cycloalkyl group, an aryl group, or an aralkyl group.)
or a salt thereof for manufacturing a frataxin enhancer.

9. The use of a compound represented by the following Formula (I): 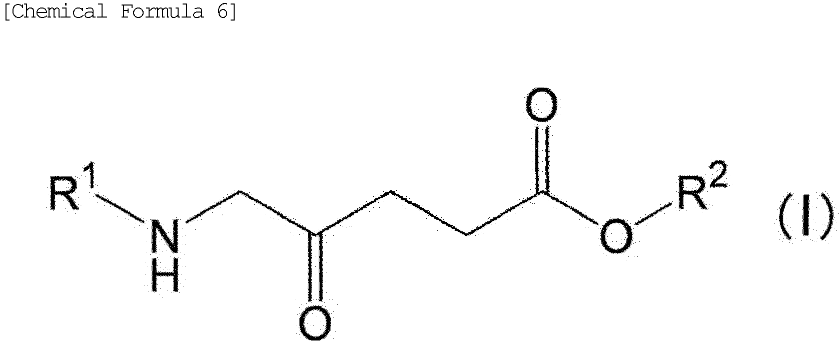 (wherein R¹ represents a hydrogen atom or an acyl group, and R² represents a hydrogen atom, a linear or branched alkyl group, a cycloalkyl group, an aryl group, or an aralkyl group.)
or a salt thereof for manufacturing a therapeutic and/or prophylactic agent for a disease caused by reduction in frataxin production.
